# EUROPEAN PATENT APPLICATION

(11) **EP 4 349 442 A1**
(43) Date of publication of application: **10.04.2024**
(21) Application number: 22816112.1
(22) Date of filing: 31.05.2022
(51) Int. Cl.: B01D 9/02, C07C 51/43, C07C 57/055, B01F 27/86

(54) **TANK USED IN REFINING DEVICE**

(30) Priority: 02.06.2021 JP 2021093028
(71) Applicant: Nippon Shokubai Co., Ltd., Osaka-shi, Osaka 541-0043 (JP)
(72) Inventor: FUKUMOTO, Toshiya, Himeji-shi, Hyogo 671-1282 (JP); TAKEMOTO, Yasutaka, Himeji-shi, Hyogo 671-1282 (JP); MUKAE, Masashi, Himeji-shi, Hyogo 671-1282 (JP)
(74) Representative: Schön, Christoph
(86) International application number: PCT/JP2022/022143
(87) International publication number: WO 2022/255366

(57) **Abstract**

The present invention provides a method for obtaining a high-quality product. The present invention relates to a tank for use in a purification apparatus, the tank being at least one of a crystallization tank that forms a slurry containing crystals of a compound or a ripening tank that is capable of keeping crystals of a compound suspended in the ripening tank, the tank including: an agitator; and a baffle for generating an upward flow from a swirling flow, the tank being capable of forming a supernatant part consisting of a supernatant and a suspension part where the crystals of a compound are suspended.

## Description

### TECHNICAL FIELD

The present invention relates to a tank for use in a purification apparatus. Specifically, the present invention relates to a tank for use in a purification apparatus, a purification apparatus, a method for producing a compound, and a method for purifying a compound.

### BACKGROUND ART

Purification apparatuses have been widely used industrially to purify compounds that are used as raw materials for resins, for example. In many fields of the chemical industry, high-quality compounds with reduced impurities have been required, and for such compounds, various better purification apparatuses have been studies.

Industrially, many of crude compounds, which are compounds before purification, are purified through continuous purification processes. Disclosed is a method for producing acrylic acid including: collecting and crystallization purifying a gas containing acrylic acid obtained by catalytic vapor phase oxidation of a raw material gas; and returning acrylic acid obtained by decomposing a substance obtained by Michael addition of acrylic acid in a residual mother liquor to the collecting step, for example (see, for example, Patent Literature 1).

In order to obtain a high-purity compound in a higher yield, the purification uses a tank that forms a slurry containing crystals of a compound (crystallization tank) and/or a tank that grows the crystals of a compound (ripening tank).

Patent Literatures 2 to 4 disclose conventional purification methods using a crystallization tank and/or a ripening tank.

### CITATION LIST

### - Patent Literature

Patent Literature 1: JP 2007-182437 A
Patent Literature 2: JP 2005-28214 A
Patent Literature 3: JP 2012-140471 A
Patent Literature 4: JP 2002-204937 A

### SUMMARY OF INVENTION

### - Technical Problem

As described above, better purification apparatuses for producing compounds have been desired, and obtaining high-quality products (compounds) has been desired. The present invention has been made in view of the above-mentioned current state of the art, and aims to provide a method for obtaining a high-quality product.

### - Solution to Problem

The present inventors have studied a purification apparatus and focused on a tank for use in the purification apparatus. Specifically, the present inventors focused on a tank such as a crystallization tank that forms a slurry containing crystals of a compound or a ripening tank that is capable of keeping crystals of a compound suspended in the ripening tank, the tank including an agitator and a baffle for generating an upward flow from a swirling flow and capable of forming a supernatant part consisting of a supernatant and a suspension part where crystals of a compound are suspended. The present inventors found that in such a tank, the crystals of a compound can be kept sufficiently uniformly suspended for a certain period of time, the crystals can be suitably grown, and a high-quality product can be obtained. Thereby, the present invention has been achieved.

That is, the present invention relates to a tank for use in a purification apparatus, the tank being at least one of a crystallization tank that forms a slurry containing crystals of a compound or a ripening tank that is capable of keeping crystals of a compound suspended in the ripening tank, the tank including: an agitator; and a baffle for generating an upward flow from a swirling flow, the tank being capable of forming a supernatant part consisting of a supernatant and a suspension part where the crystals of a compound are suspended.

Patent Literatures 2 and 4 disclose baffles. The baffle disclosed in Patent Literature 2 basically dams the upward flow, and does not keep the suspension state by generating an upward flow. Also, in the agitation vessel disclosed in Patent Literature 4, the crystals are not mixed uniformly in the suspension part and coarse crystals deposit on the bottom surface, which may cause mixing of the crystals into the supernatant part.

### - Advantageous Effects of Invention

Use of the tank of the present invention can provide a high-quality product.

### BRIEF DESCRIPTION OF DRAWINGS

FIG. 1 is a schematic cross-sectional side view of an exemplary tank of the present invention.
FIG. 2 is a schematic cross-sectional side view of another exemplary tank of the present invention.
FIG. 3 is a schematic cross-sectional side view of another exemplary tank of the present invention.
FIG. 4 is a schematic cross-sectional side view of an exemplary separation mechanism (reservoir) for the tank of the present invention shown in FIG. 3.
FIG. 5 is a schematic cross-sectional view of an exemplary tank of the present invention viewed from an upper side (a top-roof side).
FIG. 6 is a schematic cross-sectional side view of another exemplary separation mechanism for the tank of the present invention.
FIG. 7 is a schematic cross-sectional side view of another exemplary separation mechanism for the tank of the present invention.
FIG. 8 is a schematic cross-sectional side view of another exemplary separation mechanism for the tank of the present invention.

### DESCRIPTION OF EMBODIMENTS

The present invention will be described in detail below.

It should be noted that combinations of two or more of the preferred features of the present invention described below are also preferred embodiments of the present invention.

The following first describes a tank of the present invention (crystallization tank and/or ripening tank for use in a purification apparatus), followed by descriptions of a purification apparatus of the present invention, a method for producing a compound of the present invention, and a method for purifying a compound of the present invention in this order.

### (Tank for use in purification apparatus)

The tank of the present invention includes at least one of a crystallization tank that forms a slurry containing crystals of a compound or a ripening tank that is capable of keeping crystals of a compound suspended in the ripening tank, the tank including: an agitator; and a baffle for generating an upward flow from a swirling flow, the tank being capable of forming a supernatant part consisting of a supernatant and a suspension part where the crystals of a compound are suspended.

During use of the tank, a suspended state can be formed in a portion of the tank by appropriately generating an upward flow from the swirling flow.

Thereby, in the suspension part in the tank, crystals can be kept for a certain period of time, and after the crystals have grown sufficiently, they can be withdrawn as a slurry or the like from a portion in the vicinity of the bottom of the tank, for example. For example, in the ripening tank, when the crystals are kept for a certain period of time, fine crystals are melted by Ostwald ripening and large crystals grow further, so that the crystal size distribution becomes narrow. As a result, high-quality crystals can be obtained, and when such crystals are subjected to the subsequent purification in the wash column, for example, the purification efficiency in the wash column can be further improved. Even in the crystallization tank, the same effect as in the ripening tank can be expected by keeping the crystals for a certain period of time.

The retention time of the compound in the ripening tank may be appropriately adjusted according to the type of the compound to be purified. The retention time is preferably 0.5 to 6 hours in order to adjust the particle size distribution of the slurry to be sent to the wash column and to reduce the reflux ratio (flow rate of washing liquid/flow rate of compound to be purified) in the wash column. When the compound is a (meth)acrylic acid, the retention time is more preferably 1 to 5 hours, still more preferably 1.2 to 4.5 hours.

The retention time is a value calculated by dividing the volume of the suspension part in the ripening tank by the flow rate of the slurry fed from the ripening tank to the wash column in the next step (subsequent stage).

The size of the crystallization tank is determined in consideration of the retention time and the required heat transfer area. The retention time of the compound in the crystallization tank depends on the operating conditions.

The agitator may be any one that can sufficiently generate the swirling flow in the tank. Any known agitator can be used, and the agitator may be an agitator, a mixer, a blender, a kneader, or another agitator. The agitator may be made of any material, and preferred examples of the material include metals such as stainless steel.

When the agitator has an agitator blade, the length of the agitator blade (the distance from the agitator shaft to a tip of the blade) is preferably 1/10 or more, more preferably 1/8 or more, of the inner diameter of the tank of the present invention (herein also referred to as a tank diameter of the tank of the present invention) when the tank of the present invention is viewed from the upper side.

The length of the agitator blade is typically 1/2 or less of the inner diameter of the tank of the present invention.

Multiple agitator blades may be present in the axial direction. In other words, the agitator may have a multi-stage blade. When multiple agitator blades are present in the axial direction, any one of the agitator blades preferably has a length of which the ratio relative to the inner diameter of the tank falls within the above-described preferred range. More preferably, all the agitator blades have a length of which the ratio relative to the inner diameter of the tank falls within the above-described preferred range.

Herein, the inner diameter of the tank refers to the inner diameter of the tank of the present invention when the tank is viewed from the upper side. When the tank has a shape different from a cylindrical shape, the inner diameter of the tank refers to the maximum distance (distance on the horizontal plane) between two points on the contour corresponding to the inner wall surface of the tank when the tank of the present invention is viewed from the upper side.

The baffle for generating an upward flow from the swirling flow is capable of changing at least a portion of the horizontal flow in the swirling flow into an upward flow when the swirling flow collides with the baffle. The swirling flow is generated, for example, by agitating the slurry containing crystals of a compound in the tank using the agitator.

Here, the tank of the present invention is not limited to the tank in use. In other words, the tank of the present invention should be one capable of forming a supernatant part consisting of a supernatant and a suspension part where crystals of a compound are suspended. The baffle should be one capable of generating an upward flow from the swirling flow by performing agitation with an agitator, for example, during use of the tank of the present invention.

The baffle should be provided in the tank so as to generate an upward flow from the swirling flow. For example, the baffle is preferably provided along the direction from the top-roof side to the bottom side of the tank.

When the baffle is provided along the direction from the top-roof side to the bottom side of the tank, the baffle does not have to be in contact with the top roof or the bottom of the tank, and the inclination in the longitudinal direction of the baffle should correspond to the direction from the top-roof side to the bottom side of the tank.

In particular, the baffle is preferably provided such that the longitudinal direction thereof forms with the vertical direction an angle within the range of 0° to 30°, more preferably within the range of 0° to 15°, further preferably within the range of 0° to 10°, particularly preferably within the range of 0° to 5°. Most preferably, the baffle is provided in the vertical direction.

The longitudinal direction of the baffle refers to the direction of the shortest line segment that connects the highest point (top) to the lowest point (bottom) of the baffle.

As described above, the tank of the present invention should be one capable of forming a supernatant part (herein also referred to as a clarification part) consisting of a supernatant and a suspension part where crystals of a compound are suspended, during use of the tank.

For example, the baffle provided on the bottom side of the tank and along the direction from the top-roof side (upper side) to the bottom side of the tank moderately generates an upward flow from the swirling flow on the bottom side of the tank during use of the tank. Thereby, a suspended state can be formed on the bottom side of the tank and a supernatant part consisting of a mother liquor (supernatant) is formed on the upper side of the tank.

Thus, if necessary, the mother liquor derived from the slurry can be very suitably recovered from the supernatant part.

Herein, the supernatant part is a part where the mother liquor (supernatant) derived from the slurry is present during use of the tank of the present invention, and the suspension part is a part where the slurry containing crystals of a compound (suspension) is present during use of the tank of the present invention.

Preferably, the baffle is provided on the bottom side of the tank.

The expression "the baffle is provided on the bottom side of the tank" refers to that the position in height of the center of gravity of the baffle is located in the lower half of the inner volume of the tank.

In particular, the baffle preferably creates a supernatant part consisting of a supernatant and a suspension part where crystals of a compound are suspended, with the boundary between the parts corresponding to the position in height of the top of the baffle.

For example, when the baffle is provided in the vertical direction on the bottom side of the tank, the baffle typically can create a supernatant part consisting of a supernatant and a suspension part where crystals of a compound are suspended, with the boundary between the parts corresponding to the position in height of the top of the baffle.

In the tank of the present invention, the baffle is preferably provided in the vicinity of the inner wall surface of the tank.

The phrase "the baffle is provided in the vicinity of the inner wall surface of the tank" refers to that when the tank of the present invention is viewed from the upper side, the shortest distance between the baffle and the inner wall surface of the tank is 1/5 or less of the inner diameter of the tank of the present invention. The shortest distance is preferably 1/10 or less, more preferably 1/20 or less, of the inner diameter of the tank of the present invention.

The baffle may be in contact with the inner wall surface of the tank of the present invention.
Preferably, the shortest distance between the baffle and the inner wall surface of the tank is 1/100 or more of the inner diameter of the tank of the present invention, for example.

More preferably, the baffle is provided in the direction from the inner wall surface of the tank of the present invention toward the center of the tank.

For example, the width of the baffle is preferably 1/30 or more, more preferably 1/20 or more, still more preferably 1/18 or more, particularly preferably 1/15 or more, of the inner diameter of the tank of the present invention.

The width of the baffle is preferably 1/2 or less, more preferably 1/5 or less, still more preferably 1/8 or less, of the inner diameter of the tank of the present invention.

The height of the baffle is preferably 1/5 or more, more preferably 3/10 or more, still more preferably 2/5 or more, particularly preferably 1/2 or more, of the height of the cylindrical part of the tank body of the tank of the present invention, for example.

The height of the baffle is preferably 9/10 or less, more preferably 4/5 or less, of the height of the cylindrical part of the tank body.

The cylindrical part of the tank body of the tank of the present invention is a columnar portion having a constant inner diameter of the tank of the present invention, and is preferably a portion having a maximum inner diameter. Also, the cylindrical part of the tank body is preferably a cylindrical portion of the tank of the present invention.

The height of the baffle refers to the difference between the position in height of the top of the baffle and the position in height of the bottom of the baffle.

When the tank of the present invention includes multiple baffles, preferably, any one of the baffles should satisfy the above-mentioned ratio of the height of the baffle to the height of the cylindrical part of the tank body. More preferably, all of the baffles satisfy the above-mentioned ratio of the height of the baffle to the height of the cylindrical part of the tank body.

During use of the tank of the present invention, as described above, the upper side of the top of the baffle corresponds to the supernatant part, and the lower side of the top of the baffle corresponds to the suspension part, with the boundary between the parts corresponding to the position in height of the top of the baffle. Thus, the volume ratio (the proportions of the volumes) between the supernatant part and the suspension part during use of the tank of the present invention can be adjusted by adjusting the ratio of the height of the baffle to the height of the cylindrical part of the tank body.

The inner diameter of the tank of the present invention is preferably 1/10 or more, more preferably 1/8 or more, still more preferably 1/6 or more, particularly preferably 1/4 or more, of the height of the cylindrical part of the tank body.

The inner diameter of the tank of the present invention is preferably 1 or less, more preferably 1/1.2 or less, still more preferably 1/1.5 or less, particularly preferably 1/1.8 or less, of the height of the cylindrical part of the tank body.

The tank of the present invention is preferably configured such that the volume of the suspension part is 1/5 or more, more preferably 3/10 or more, still more preferably 2/5 or more, particularly preferably 1/2 or more, of the volume of the entire content (supernatant part and suspension part) kept in the tank.

Preferably, the tank of the present invention is preferably configured such that the volume of the suspension part is 9/10 or less, more preferably 4/5 or less, of the volume of the entire content (supernatant part and suspension part) kept in the tank.

The thickness of the baffle may be any thickness that can achieve sufficient strength against collisions of the swirling flow, and is preferably 1 to 100 mm, for example.

Examples of a material for the baffle include metals such as stainless steel and resins.

Multiple baffles may be provided in the tank of the present invention. Preferably, multiple baffles are provided. When multiple baffles are provided in the tank of the present invention, the baffles are, for example, preferably symmetrically arranged with respect to the center of the tank of the present invention when the tank of the present invention is viewed from the upper side.

Preferably, the tank of the present invention includes, above a top of the baffle: a feed port for feeding a slurry containing crystals of a compound to the tank; a withdrawal port for withdrawing a mother liquor from the tank; and a partition provided between the feed port and the withdrawal port along a direction from a top-roof side to a bottom side of the tank.

The expression "a partition provided between the feed port and the withdrawal port" refers to that the partition is provided to separate the feed port and the withdrawal port when the tank of the present invention is viewed from the upper side and/or a lateral side.

Preferably, the partition is provided closer to the feed port than the withdrawal port when the tank of the present invention is viewed from the upper side.

When the tank of the present invention is viewed from the upper side, the partition and the inner wall surface of the tank preferably define a region where the feed port is present and a region where the withdrawal port is present. In this case, the partition is preferably provided such that the ratio of the area of the region where the withdrawal port is present to the area of the region where the feed port is present is preferably 1 or more, more preferably 2 or more, still more preferably 3 or more, particularly preferably 3.5 or more.

The area ratio is preferably 20 or less, for example, from the viewpoint of suitably arranging the feed port.

When the partition has an inclination and the area ratio varies with a position in height of the partition, the area ratio should be within the above-mentioned preferred range at at least one position in height of the partition, and the area ratio is preferably within the above-mentioned preferred range at all the positions in height of the partition.

When the tank of the present invention is viewed from the upper side, more preferably, the cross-section of the tank is divided by the partition and the inner wall surface of the tank into a region where the feed port is present and a region where the withdrawal port is present, and the partition is orthogonal to one of the inner diameters of the tank of the present invention. In this case, the ratio of the length of an inner-diameter portion in the region where the withdrawal port is present to the length of an inner-diameter portion in the region where the feed port is present is preferably 1 or more, more preferably 2 or more, still more preferably 2.5 or more.

The upper limit of the length ratio is not limited, and may be 10 or less, for example.

When the partition has an inclination and the length ratio varies with a position in height of the partition, the length ratio should be within the above-mentioned preferred range at at least one position in height of the partition, and the length ratio is preferably within the above-mentioned preferred range at all the positions in height of the partition.

When the tank of the present invention is viewed from the upper side, the distance between the withdrawal port and the feed port is preferably 2/5 or more, more preferably 1/2 or more, still more preferably 3/5 or more, of the inner diameter of the tank of the present invention.

The upper limit of the distance is not limited, and is typically equal to or less than the inner diameter of the tank of the present invention. When the tank includes multiple withdrawal ports and/or multiple feed ports, the distance between one of the withdrawal ports and one of the feed ports preferably falls within the range of the ratio described above. More preferably, all of the distances between the withdrawal ports and the feed ports fall within the range of the ratio described above.

When the partition is provided along the direction from the top-roof side to the bottom side of the tank, the partition does not have to be in contact with the top roof or the bottom of the tank, and the inclination in the longitudinal direction of the partition should correspond to the direction from the top-roof side to the bottom side of the tank.

In particular, the partition is preferably provided such that the longitudinal direction thereof forms with the vertical direction an angle within the range of 0° to 30°, more preferably within the range of 0° to 15°, further preferably within the range of 0° to 10°, particularly preferably within the range of 0° to 5°. Most preferably, the partition is provided in the vertical direction.

The longitudinal direction of the partition refers to the direction of the shortest line segment that connects the highest point (top) to the lowest point (bottom) of the partition.

The width of the partition is preferably 1/5 or more, more preferably 2/5 or more, still more preferably 3/5 or more, particularly preferably 4/5 or more, of the inner diameter of the tank of the present invention, for example.

The width of the partition is typically equal to or less than the inner diameter of the tank of the present invention, and is preferably 19/20 or less of the inner diameter of the tank, for example.

The height of the partition is preferably 1/20 or more, more preferably 1/10 or more, still more preferably 1/8 or more, of the height of the cylindrical part of the tank body.

The height of the partition is preferably 3/5 or less, more preferably 2/5 or less, of the height of the cylindrical part of the tank body.

The width and height of the partition can be appropriately selected according to the size of the tank as described above.

The thickness of the partition may be any thickness that can achieve sufficient strength, and is preferably 1 to 100 mm, for example.

Examples of a material for the partition include metals such as stainless steel and resins.

The tank may include multiple partitions, and preferably includes only one partition.

The partition is preferably provided with respect to the baffle at an angle within the range of 0° to 30°, more preferably within the range of 0° to 15°, still more preferably within the range of 0° to 10°, particularly preferably within the range of 0° to 5° when viewed from the upper side.

Most preferably, in the tank of the present invention, the partition is provided in parallel to the baffle.

Thereby, the partition is prevented from acting like a baffle (generating an upward flow from the swirling flow) during use of the tank, and crystals can be further sufficiently prevented from mixing the supernatant part.

The feed port, the withdrawal port, and the partition are provided above the top of the baffle. In other words, the feed port, the withdrawal port, and the partition are provided in the supernatant part.

In order to further prevent crystals from mixing into the mother liquor to be withdrawn from the supernatant, the vertical distance between the partition and the baffle is preferably 1/20 or more, more preferably 1/18 or more, still more preferably 1/15 or more, particularly preferably 1/10 or more, of the height of the cylindrical part of the tank body. Also in such a structure, the partition is prevented from acting like a baffle, and a significant effect of preventing crystals from mixing the supernatant part can be achieved.

The upper limit of the vertical distance between the partition and the baffle to the height of the cylindrical portion of the tank body is not limited, and is typically 4/5 or less.

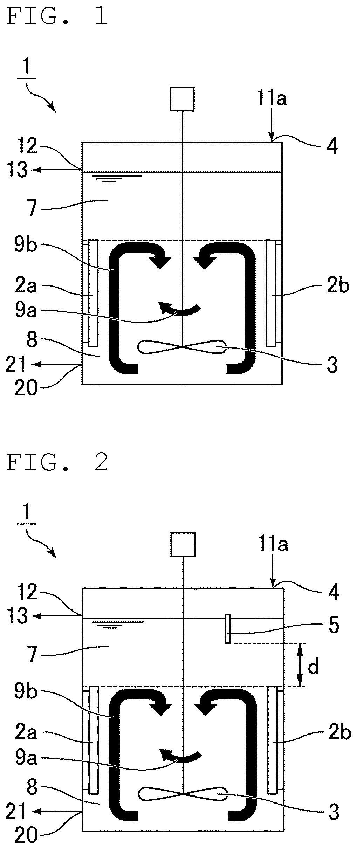

Here, the vertical distance between the partition and the baffle is the minimum vertical distance between the position where the partition is present and the position where the baffle is present. Typically, the vertical distance is the difference between the position in height of the bottom of the partition and the position in height of the top of the baffle (e.g., the vertical distance d shown in FIG. 2).

When the tank includes multiple partitions and/or baffles, the vertical distance between one of the partitions and one of the baffles preferably falls within the range of the ratio described above. More preferably, all of the vertical distances between the partitions and the baffles fall within the range of the ratio described above.

The feed port and the baffle are preferably spaced apart from each other in the horizontal direction as much as possible. The distance between the feed port and the baffle is preferably 1/20 or more, more preferably 1/10 or more, still more preferably 1/8 or more, still further more preferably 1/5 or more, particularly preferably 2/5 or more, of the tank diameter when the tank of the present invention is viewed from the upper side.

The upper limit of the distance between the feed port and the baffle relative to the tank diameter is not limited, and is typically 9/10 or less.

Here, as described above, the distance between the feed port and the baffle is the shortest horizontal distance between the feed port and the baffle when the tank of the present invention is viewed from the upper side.

When the tank includes multiple feed ports and/or multiple baffles, the distance between one of the feed ports and one of the baffles preferably falls within the range of the ratio described above. More preferably, all of the distances between the feed ports and the baffles fall within the range of the ratio described above.

Herein, the term "above the top of the baffle" refers to a position higher than the position in height of the top of the baffle, and is not limited to over the baffle.

Although FIG. 1, which is described later, shows a tank including one feed port and one withdrawal port, the tank may include multiple feed ports and/or multiple withdrawal ports. For example, the tank may include 3 to 12 feed ports. The nozzle constituting the feed port may be bent at its tip to feed the slurry along the inner wall surface of the tank, or may be placed so that the tip is immersed in the liquid to feed the slurry into the liquid.

Preferably, the tank of the present invention includes, above a top of the baffle: a withdrawal port for withdrawing a mother liquor from the tank; and a separation mechanism including a weir for overflowing the supernatant and flows the supernatant into the withdrawal port so as to prevent the crystals of a compound from mixing into the mother liquor.

When the tank of the present invention includes the separation mechanism, crystals can be sufficiently prevented from entering the withdrawal port, and the mother liquor can be withdrawn suitably.

The separation mechanism is typically composed of a bottom and a weir (lateral-side part) so as to cover the withdrawal port from the bottom side and a lateral side of the tank of the present invention. A part of the weir can be replaced by the inner wall surface of the tank. The top of the weir (lateral-side part) may have a notch. The notch has any shape, and preferably has a triangular shape (inverted triangular shape) or a rectangular shape, for example, when the weir is viewed from a lateral side.

The upper side of the separation mechanism is partially or completely uncovered, and the mother liquor overflowing the weir can be introduced into the separation mechanism through the upper side.

The bottom of the separation mechanism may be horizontal, or is preferably inclined at an angle of, for example, 0.5° to 30° with respect to the horizontal direction so that the position in height of the bottom decreases toward the withdrawal port. Thereby, the mother liquor in the separation mechanism can be suitably flowed through the withdrawal port.

The sizes and materials of the bottom and the weir can be designed and selected as appropriate.

As described above, the tank of the present invention preferably includes a withdrawal port for withdrawing the mother liquor from the tank.

Preferably, the tank of the present invention includes, above the withdrawal port for withdrawing the mother liquor from the tank: an extra withdrawal port for withdrawing the mother liquor from the tank, the extra withdrawal port being provided such that a position in height of a lower end of an opening of the extra withdrawal port is equal to or lower than a position in height of a top of the weir in the separation mechanism.

The extra withdrawal port is intended to be used in place of the withdrawal port for withdrawing the mother liquor from the tank when the withdrawal port is clogged to be unusable, for example. The extra withdrawal port can withdraw the mother liquor that can be held in the separation mechanism.

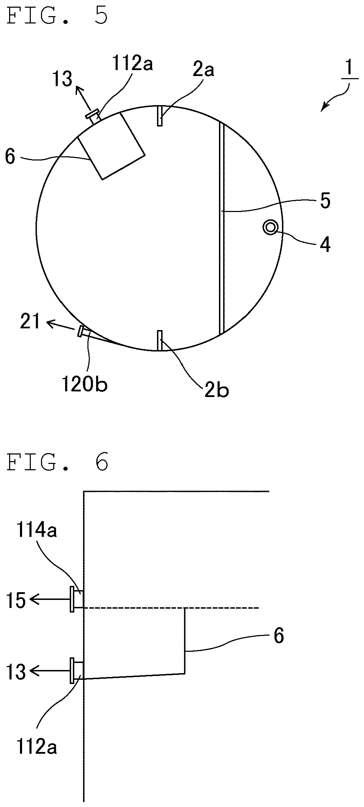

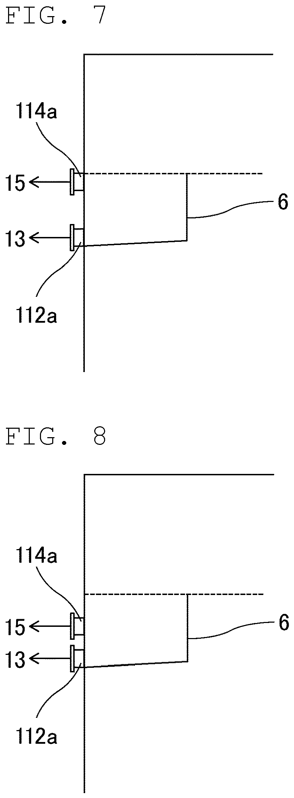

As described above, the extra withdrawal port should be provided above the withdrawal port for withdrawing the mother liquor from the tank and such that the position in height of the lower end of the opening of the extra withdrawal port is equal to or lower than the position in height of the top of the weir in the separation mechanism. The position in height of the extra withdrawal port can be adjusted within the range. Examples of the position in height of the extra withdrawal port are shown in FIGS. 6 to 8. In FIGS. 6 to 8, the position in height of the top of the weir in the separation mechanism is indicated by a broken line. The position in height of the lower end of the opening of the nozzle 114a constituting the extra withdrawal port shown in FIG. 6 is the same as the position in height of the top of the weir in the separation mechanism. Further, the position in height of the lower end of the opening of the nozzle 114a constituting the extra withdrawal port shown in FIGS. 7 and 8 is lower than the position in height of the top of the weir in the separation mechanism.

The separation mechanism and the baffle are preferably spaced apart from each other in the horizontal direction as much as possible. When the tank of the present invention is viewed from the upper side, the distance between the separation mechanism and the baffle is preferably 1/50 or more, more preferably 1/40 or more, still more preferably 1/30 or more, further more preferably 1/20 or more, particularly preferably 1/15 or more, of the tank diameter.

The upper limit of the distance between the separation mechanism and the baffle relative to the tank diameter is not limited, and the distance is typically 9/10 or less.

The distance between the separation mechanism and the baffle is the shortest horizontal distance between the separation mechanism and the baffle when the tank of the present invention is viewed from the upper side.

When the tank includes multiple separation mechanisms and/or multiple baffles, the distance between one of the separation mechanisms and one of the baffles preferably falls within the range of the ratio described above. More preferably, all of the distances between the separation mechanisms and the baffles fall within the range of the ratio described above.

Preferably, the tank of the present invention includes, below the top of the baffle: a withdrawal port for withdrawing the slurry containing crystals of a compound from the tank, the withdrawal port being provided so as to withdraw the slurry containing crystals of a compound along a tangential direction of a contour line corresponding to an inner wall surface of the tank when the tank is viewed from an upper side.

The position "below the top of the baffle" may be any position lower than the position in height of the top of the baffle, and is not limited to below the baffle. The withdrawal port is preferably provided at a position lower than the center of gravity of the baffle, and more preferably provided at a position equal to or lower than the position in height of the bottom of the baffle.

The expression "the withdrawal port being provided so as to withdraw the slurry containing crystals of a compound along a tangential direction of a contour line corresponding to an inner wall surface of the tank when the tank is viewed from an upper side" refers to that the nozzle or line constituting the withdrawal port is provided within the range of 15° or less with respect to the tangential direction of the contour line corresponding to the inner wall surface of the tank. The nozzle or line is more preferably provided within the range of 10° or less, still more preferably within the range of 5° or less, with respect to the tangential direction, and particularly preferably provided in parallel to the tangential direction.

When the withdrawal port is provided in this manner, the slurry containing crystals of a compound can be efficiently withdrawn by utilizing the swirling flow. Also, the crystals can be sufficiently prevented from depositing on the bottom surface of the tank.

The withdrawal port for withdrawing the slurry and the baffle are preferably spaced apart from each other in the horizontal direction as much as possible. When the tank of the present invention is viewed from the upper side, the distance between the withdrawal port and the baffle is preferably 1/30 or more, more preferably 1/20 or more, of the tank diameter.

The upper limit of the distance between the withdrawal port and the baffle relative to the tank diameter is not limited, and is typically 9/10 or less.

Here, the distance between the withdrawal port and the baffle is the shortest horizontal distance between the withdrawal port and the baffle when the tank of the present invention is viewed from the upper side.

The slurry may be withdrawn using a pump, for example. Preferred examples of the pump include a centrifugal pump, a diaphragm pump, and a rotary pump.

When the shape of the bottom surface of the tank is flat, crystals may accumulate in the corners depending on the agitation conditions. Thus, the corners may be chamfered or rounded.

The tank of the present invention may have any size. For example, the tank preferably has an inner diameter of 100 to 50000 mm. The tank preferably has a height of 200 to 100000 mm.

Instruments such as a thermometer, a pressure gauge, a liquid level gauge (e.g., of radar type), and a level switch (e.g., of float type) may be provide in the main body or periphery of the crystallization tank or the ripening tank of the present invention. The ripening tank may include a sight glass (an observation window) in its side wall or the like. The sight glass may be covered with a cover. The ripening tank may include a hole such as a manhole or a hand hole (a hole for inserting a hand for maintenance) in its top roof, side wall, or the like. The ripening tank may include a rupture device or the like in its top roof or the like. The numbers of these are not limited.

FIG. 1 is a schematic cross-sectional side view of an exemplary tank of the present invention. A crystal-containing slurry 11a is supplied into a tank 1 via a nozzle 4. Subsequently, the agitator shaft of the agitator in the tank 1 is rotated in an agitating direction 9a, and the crystal-containing slurry is agitated by an agitator blade 3. Thereby, a horizontal swirling flow generates, collides with baffles 2a and 2b to become an upward flow. By generating an upward flow in a flow 9b of the crystal-containing slurry in this manner, a suspension part 8 suitably becomes suspended, and the crystal-containing slurry can be kept in the suspension part 8 for a certain period of time for growing crystals in the slurry. Thereafter, a crystal-containing slurry 21 can be withdrawn from, for example, a portion in the vicinity of the bottom of the tank through a withdrawal port 20. In a supernatant part 7, a mother liquor 13 can be withdrawn from a mother liquor withdrawal port 12 and reused.

In FIG. 1, the boundary between the supernatant part 7 and the suspension part 8 is shown by a broken line.

FIG. 2 is a schematic cross-sectional view of another exemplary tank of the present invention. In FIG. 2, a partition 5 may be provided in the supernatant part 7 to separate the nozzle 4, which is a slurry feed port, from the mother-liquor withdrawal port 12, thereby preventing crystals from entering the mother-liquor withdrawal port 12.

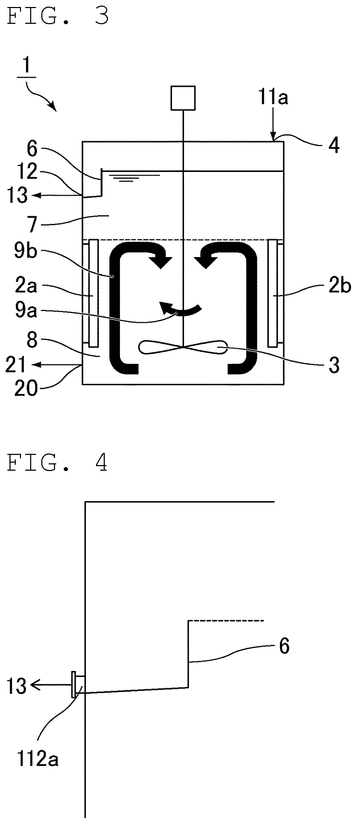

FIG. 3 is a schematic cross-sectional side view of another exemplary tank of the present invention. In FIG. 3, the mother liquor withdrawal port 12 is covered with a separation mechanism 6, and crystals can be prevented from entering the mother liquor withdrawal port 12. In addition, the mother liquor 13 can be suitably withdrawn through the mother liquor withdrawal port 12.

FIG. 4 is a schematic cross-sectional side view of an exemplary separation mechanism (reservoir) for the tank of the present invention shown in FIG. 3. A nozzle 112a, which constitutes the mother liquor withdrawal port provided in the supernatant part of the tank, is covered with the separation mechanism 6 composed of a weir and a bottom, thereby sufficiently preventing crystals from entering the nozzle 112a. The bottom of the separation mechanism 6 may be inclined such that the position in height thereof decreases toward the nozzle 112a. Thereby, the mother liquor in the separation mechanism 6 can be flowed into the nozzle 112a very suitably.

FIG. 5 is a schematic cross-sectional view of an exemplary tank of the present invention viewed from an upper side (a top-roof side). As shown in FIG. 5, a nozzle 120b constituting the withdrawal port is provided such that the crystal-containing slurry 21 is withdrawn in parallel to the tangential direction of the contour line corresponding to the inner wall surface of the tank when the tank 1 is viewed from the upper side. Thereby, the slurry containing crystals of a compound can be efficiently withdrawn by utilizing the swirling flow. Also, the crystals can be sufficiently prevented from depositing on the bottom surface of the tank.

FIGS. 6 to 8 are each a schematic cross-sectional side view of another exemplary separation mechanism for the tank of the present invention. In FIGS. 6 to 8, the tank further includes the nozzle 114a constituting the extra withdrawal port for withdrawing the mother liquor from the tank, above the nozzle 112a constituting the withdrawal port for withdrawing the mother liquor from the tank. The nozzle 114a is located above the nozzle 112a, and the position in height of the lower end of the opening of the nozzle 114a should be equal to or lower than the position in height of the top of the weir in the separation mechanism. The position in height of the nozzle 114a can be adjusted as appropriate within the range. For example, as described above, the position in height of the lower end of the opening of the nozzle 114a constituting the extra withdrawal port shown in FIG. 6 is the same as the position in height of the top of the weir in the separation mechanism. Further, the position in height of the lower end of the opening of the nozzle 114a constituting the extra withdrawal port shown in FIGS. 7 and 8 is lower than the position in height of the top of the weir in the separation mechanism.

Thereby, when the mother liquor 13 cannot be withdrawn through the nozzle 112a due to, for example, clogging of the nozzle 112a, the mother liquor 15 can be suitably withdrawn through the nozzle 114a.

### (Purification apparatus of the present invention)

The present invention also relates to a purification apparatus including the tank of the present invention.

The purification apparatus of the present invention is preferably one capable of performing continuous purification. For example, the purification apparatus further includes a ripening tank which is the tank of the present invention, a crystallization tank as the preceding stage of the ripening tank, and a wash column (preferably a wash column that forcibly transports crystals) as the subsequent stage of the ripening tank in the present invention.

When the purification apparatus of the present invention further includes the crystallization tank, the purification apparatus of the present invention may include one or multiple crystallization tanks. When the purification apparatus of the present invention includes multiple crystallization tanks (first to N-th tanks), these crystallization tanks are preferably connected in series. In this case, the purification apparatus of the present invention typically includes a line for sending the slurry containing crystals of a compound from one crystallization tank to another crystallization tank optionally via a solid-liquid separator. In this case, in the purification apparatus of the present invention, at least one crystallization tank includes a line for feeding a liquid to be purified containing a compound. Preferably, in the purification apparatus of the present invention, at least the N-th crystallization tank includes a line for feeding the slurry containing crystals of a compound to the ripening tank.

When the purification apparatus of the present invention includes a crystallization tank and a ripening tank, at least one of the crystallization tank or the ripening tank is required to be the tank of the present invention.

When the purification apparatus of the present invention further includes the wash column, the purification apparatus of the present invention preferably includes a line for feeding a slurry containing crystals of a compound from the tank of the present invention to the wash column.

Preferably, the purification apparatus of the present invention further includes a line for sending out the product from the wash column.

The purification apparatus of the present invention may further include a line for returning the mother liquor from a tank or unit in a subsequent stage to a tank or unit in a preceding stage.

The purification apparatus of the present invention may further include a mechanism that controls the amount of the slurry to be sent and the amount of the mother liquor to be returned. Examples of the control mechanism include valves attached to the lines.

The purification apparatus of the present invention may appropriately include different units commonly used in the purification apparatus.

### (Method for producing compound of the present invention)

The present invention also relates to a method for producing a compound, the method including: feeding a slurry containing crystals of a compound to a tank; agitating the slurry containing crystals of a compound in the tank; and withdrawing the slurry containing crystals of a compound agitated in the agitating from the tank, the tank being at least one of a crystallization tank that forms a slurry containing crystals of a compound or a ripening tank that is capable of keeping crystals of a compound suspended in the ripening tank, the tank including: an agitator; and a baffle for generating an upward flow from a swirling flow, the tank being capable of forming a supernatant part consisting of a supernatant and a suspension part where the crystals of a compound are suspended.

In the method for producing a compound of the present invention, the compound is preferably a (meth)acrylic acid.

In the method for producing a compound of the present invention, the feeding step, the agitating step, and the withdrawing step are basically performed in the stated order on a substance to be purified. (For example, as shown in FIG. 1, the crystal-containing slurry 11a is fed into the tank 1 through the nozzle 4. Subsequently, the agitator shaft of the agitator in the tank 1 is rotated in the agitating direction 9a, and the crystal-containing slurry is agitated by the agitator blade 3. Thereby, a horizontal swirling flow generates, collides with the baffles 2a and 2b to become an upward flow. By generating an upward flow in the flow 9b of the crystal-containing slurry in this manner, the suspension part 8 suitably becomes suspended, and the crystal-containing slurry can be kept in the suspension part 8 for a certain period of time for growing crystals in the slurry. Thereafter, the crystal-containing slurry 21 is withdrawn from, for example, a portion in the vicinity of the bottom of the tank through the withdrawal port 20.) The following describes the feeding step, the agitating step, and the withdrawing step in the stated order, followed by descriptions of the mother-liquor withdrawing step and other steps. In the case of continuous purification, the steps are typically performed simultaneously when the tank is viewed as a whole.

Herein, the term "compound" refers to a compound obtainable by the production method of the present invention, and does not refer to raw materials, by-products, or solvents in the production method of the present invention. The term "compound" may also be referred to as a "target compound" or a "target substance". Herein, the term "impurities" refers to components other than the "compound", such as raw materials, by-products, and solvents.

### <Feeding step>

In the feeding step, the slurry containing crystals of a compound is fed to a tank. The crystal-containing slurry is a suspension of the crystals of a compound and a mother liquor. In other words, the liquid portion of the slurry containing crystals of a compound to be fed to the tank is the mother liquor. The crystal-containing slurry can be obtained by forming crystals in a compound-containing solution (e.g., a crude (meth)acrylic acid aqueous solution or a crude (meth)acrylic acid solution) as described later. The compound-containing solution may be prepared in-house or procured from outside sources. When the tank is a ripening tank, the compound-containing solution and the mother liquor returned from the next step (e.g., the wash column) may be fed to the tank (e.g., a ripening tank). The compound-containing solution encompasses a crude compound.

In order to more stably obtain a product, the mass percentage of the crystals in the crystal-containing slurry to be fed to the tank is preferably 25% by mass or more, more preferably 30% by mass or more, still more preferably 35% by mass or more.

In order to achieve excellent fluidity of the slurry and to further reduce the risk of pipe clogging, the mass percentage of the crystals is preferably 55% by mass or less, more preferably 50% by mass or less, still more preferably 45% by mass or less.

The crystal-containing slurry to be fed to the tank may be one concentrated by a solid-liquid separator, for example.

Herein, in the case of a simple "crystal-containing slurry to be fed to the tank", the crystal-containing slurry to be fed to the tank refers to the crystal-containing slurry immediately before being fed to the tank, and, for example, refers to the crystal-containing slurry in a pipe or nozzle for feeding the crystal-containing slurry to the tank.

Preferably, in the crystal-containing slurry to be fed to the tank, the mother liquor contains the compound. Examples of the mother liquor include the compound and an aqueous solution of the compound. The mother liquor typically contains impurities other than the compound and water.

In the method for producing a compound of the present invention, the purity (mass percentage) of the compound in the mother liquor in the crystal-containing slurry to be fed to the tank is preferably 99% by mass or less.

Preferably, the mass percentage of the compound in the mother liquor is 80% by mass or more.

In the production method of the present invention, the compound is preferably an easily polymerizable compound having a reactive double bond.

In particular, in the production method of the present invention, the compound is more preferably an unsaturated carboxylic acid, still more preferably a (meth)acrylic acid, particularly preferably acrylic acid. Herein, the term "(meth)acrylic acid" refers to acrylic acid and/or methacrylic acid.

In the feeding step, the crystal-containing slurry may be fed at any feed rate. For example, the feed rate is 0.2 × 10³ to 4.0 × 10⁵ kg/h in an industrial-scale tank.

In the feeding step, the feed temperature of the crystal-containing slurry can be appropriately selected according to the melting point of the compound or the like. For example, the feed temperature may be appropriately adjusted within the range of 0°C to 80°C.

For example, when the compound is a (meth)acrylic acid, the feed temperature of the crystal-containing slurry is preferably 5°C to 13°C, more preferably 6°C to 12°C.

The feed temperature of the crystal-containing slurry is the temperature of the mother liquor in the crystal-containing slurry immediately before being fed to the tank (e.g., the crystal-containing slurry in the pipe or nozzle that feeds the crystal-containing slurry to the tank).

### <Agitating step>

In the agitating step, the slurry containing crystals of a compound is agitated in the tank.

In the agitating step, the crystal-containing slurry is agitated typically using an agitator provided in the tank. Specific examples of the agitator are as described above.

In the agitating step, the rotation speed of the agitator is preferably in the range of 5 to 500 rpm, more preferably in the range of 10 to 300 rpm.

The agitating may be intermittent, preferably basically continuous during use of the tank of the present invention.

### <Withdrawing step>

In the withdrawing step, the slurry containing crystals of a compound agitated in the agitating step is withdrawn from the tank.

The mass percentage of crystals in the slurry containing crystals withdrawn from the tank is preferably 1% by mass or more, more preferably 3% by mass or more, still more preferably 5% by mass or more, particularly preferably 10% by mass or more, for example.

The mass percentage of crystals is preferably 50% by mass or less, more preferably 40% by mass or less, still more preferably 30% by mass or less.

Herein, the crystal-containing slurry or crystals withdrawn from the tank refer to the crystal-containing slurry or crystals immediately after being withdrawn from the tank, and, for example, refers to the crystal-containing slurry or crystals in the nozzle constituting the slurry withdrawal port or the withdrawal line (pipe) for withdrawing the slurry.

The crystal-containing slurry may be withdrawn from the tank at any withdrawal rate. For example, the withdrawal rate is 0.2 × 10³ to 4.0 × 10⁵ kg/h in an industrial-scale tank.

The withdrawing step can be suitably performed using a pump such as a centrifugal pump, a diaphragm pump, or a rotary pump.

The tank may be operated with its interior being under increased pressure, atmospheric pressure, or reduced pressure.

### <Mother-liquor withdrawing step>

The production method of the present invention includes withdrawing a mother liquor in the supernatant part of the tank.

The withdrawn mother liquor can be recycled and reused. The withdrawn mother liquor may be fed to a unit in a preceding stage (e.g., the crystallization tank which is the preceding stage of the ripening tank) for reuse, for example. Thereby, the quality of the compound can be further improved.

The mother liquor withdrawn in the mother-liquor withdrawing step typically contains the compound. Examples of the mother liquor include a liquid in which the compound is melted and an aqueous solution of the compound. The mother liquor typically contains impurities other than the compound and water.

The mother liquor may be withdrawn using a pump or the like.

### <Step of obtaining crystal-containing slurry>

The production method of the present invention preferably further includes a step of obtaining a slurry containing crystals of a compound from a compound-containing solution.

The compound-containing solution is preferably a crude (meth)acrylic acid aqueous solution or a crude (meth)acrylic acid solution. The crude (meth)acrylic acid aqueous solution refers to a solution in which a (meth)acrylic acid is dissolved in water and which contains impurities such as by-products produced during the production of the (meth)acrylic acid. The crude (meth)acrylic acid solution refers to a solution consisting of a (meth)acrylic acid and impurities such as by-products produced during the production of the (meth)acrylic acid. These can be obtained, for example, as follows: propylene and isobutylene are subjected to a vapor phase oxidation reaction to obtain a compound gas as a reaction product, and the compound gas is collected in an absorption column and optionally distilled. They are not limited to those synthesized in-house and may be procured from outside sources. The crude (meth)acrylic acid aqueous solution or the crude (meth)acrylic acid solution may be cooled, for example. Thereby, a slurry containing (meth)acrylic acid crystals can be obtained.

Examples of the impurities include acids such as propionic acid, acetic acid, maleic acid, benzoic acid, and acrylic acid dimers; aldehydes such as acrolein, furfural, formaldehyde, and glyoxal; acetone; and protoanemonin. In addition, solvents such as toluene and methyl isobutyl ketone may be contained.

Impurities in the compound-containing solution can be sufficiently removed by the production method of the present invention.

### <Step of obtaining compound-containing solution>

The production method of the present invention preferably further includes a step of obtaining the compound-containing solution from a raw material.

The step of obtaining the compound-containing solution may be any process that can provide the compound-containing solution. When the compound is a (meth)acrylic acid, the step of obtaining the compound-containing solution can be suitably carried out by synthesizing acrylic acid, collecting the acrylic acid, and the like, as described in JP 2007-182437 A (Patent Literature 1), for example.

In the method for producing a compound of the present invention, the (meth)acrylic acid is preferably produced from at least one raw material selected from the group consisting of propane, propylene, acrolein, isobutene, methacrolein, acetic acid, lactic acid, isopropanol, 1,3-propanediol, glycerol, and 3-hydroxypropionic acid. The (meth)acrylic acid and/or the raw material may also be biobased (meth)acrylic acids derived from renewable raw materials.

In the step of obtaining the compound-containing solution, impurities such as by-products are basically formed. For example, when the compound is a (meth)acrylic acid, the impurities generated include water; acids such as propionic acid, acetic acid, maleic acid, benzoic acid, and acrylic acid dimers; aldehydes such as acrolein, furfural, formaldehyde, and glyoxal; acetone; methyl isobutyl ketone; toluene; and protoanemonin. Such impurities can be separated with excellent efficiency by a technique such as purification using the tank in the production method of the present invention. Thereby, a product can be efficiently obtained.

### (Method for purifying compound)

The present invention also relates to a method for purifying a compound, the method including: feeding a slurry containing crystals of a compound to a tank; agitating the slurry containing crystals of a compound in the tank; and withdrawing the slurry containing crystals of a compound agitated in the agitating from the tank, the tank being at least one of a crystallization tank that forms a slurry containing crystals of a compound or a ripening tank that is capable of keeping crystals of a compound suspended in the ripening tank, the tank including: an agitator; and a baffle for generating an upward flow from a swirling flow, the tank being capable of forming a supernatant part consisting of a supernatant and a suspension part where the crystals of a compound are suspended.

The purification method of the present invention can efficiently purify a crystal-containing slurry.

Preferred embodiments of the purification method of the present invention are the same as the preferred embodiments of the production method of the present invention described above.

### EXAMPLES

The present invention will be described in more detail below with reference to examples, but the present invention is not limited by the following examples, and appropriate modifications may be made within the scope that can conform to the gist of the above and later descriptions. All of them are included in the technical scope of the present invention.

Unless otherwise specified, "%" indicates "% by mass" and "parts" indicates "parts by mass."

### (Method for preparing acrylic acid aqueous solution)

An acrylic acid aqueous solution was prepared according to the method described in WO 2010/032665 as follows: propylene was catalytically oxidized in vapor phase to obtain an acrylic acid-containing gas; and the acrylic acid-containing gas was treated in an absorption column.

### (Method for preparing feed slurry)

An acrylic acid aqueous solution was fed to the crystallization tank. A cooling medium was fed to a jacket provided around the wall of the crystallization tank for indirect cooling. The resulting crystals attached to the inner surface of the crystallization tank were scraped off with a scraper provided in the crystallization tank. Thus, a crystal-containing slurry (feed slurry) was prepared.

### (Purification apparatus/Purification conditions)

The purification apparatus used included the above-mentioned crystallization tank as a preceding stage and a ripening tank. In the purification apparatus used, the ripening tank included, as shown in FIG. 3, the baffles 2a and 2b spaced apart from the inner wall surface of the ripening tank, an extra withdrawal port above the mother liquor withdrawal port 12, and a partition between the feed port 4 for feeding the crystal-containing slurry to the ripening tank and the withdrawal port 12 for withdrawing the mother liquor from the ripening tank. Although not shown in the figures, the compound-containing solution, the mother liquor returned from the next step (e.g., the wash column), or the like may be fed to the ripening tank. The purification apparatus includes the following units:
a ripening tank 1 in which: the inner diameter (tank diameter) is 240 mm and the inner height (height of the cylindrical part of the tank body) is 480 mm,
wherein the retention time is two hours,
the slurry fed to the ripening tank 1 has a concentration (mass percentage of the crystals in the slurry) of 40%, a temperature of 10°C, and a purity of the compound in the mother liquor of 96%, and
the slurry withdrawn from the ripening tank 1 has a concentration (mass percentage of the crystals in the slurry) of 25%;
an agitator in which: the rotation speed is 131 rpm, the diameter and the length of the agitator shaft are 8 mm and 550 mm, respectively, and the blade size of the agitator blade 3 (distance from the agitator shaft to the tip of the agitator blade) is 72 mm, and the number of stages of blades is 2;
the baffles 2a and 2b in which: the height is 320 mm, the width is 18 mm, the thickness is 2 mm, the material is stainless steel, the number is 2, the installation orientation is vertical, with the horizontal distance from the inner wall surface of the tank to the baffle being 6 mm;
the partition 5 in which: the height is 96 mm, the width is 209 mm, the thickness is 2 mm, the material is stainless steel, the number is 1, the installation direction is vertical and is parallel to the baffles;
the separation mechanism 6 in which: a weir is present, the top of the weir has a notch (an inverted triangular notch when the weir is viewed from a lateral side), and the inclination of the bottom surface is 1°;
the slurry withdrawal port 20 in which: the nozzle constituting the slurry withdrawal port is provided in parallel to the tangential direction of the contour line corresponding to the inner wall surface of the ripening tank when the ripening tank is viewed from the upper side,
wherein the slurry is withdrawn by tangential withdrawing in which the slurry is withdrawn in the tangential direction of the contour line corresponding to the inner wall surface of the ripening tank when the ripening tank is viewed from the upper side; and
an extra withdrawal port (as shown in FIG. 7, the position in height of the top of the opening of the nozzle is the same as the position in height of the top of the weir in the separation mechanism).

Regarding the two regions defined by the partition and the inner wall surface of the ripening tank when the ripening tank 1 is viewed from the upper side, the ratio of the area of the region where the withdrawal port 12 for withdrawing the mother liquor from the ripening tank is present to the area of the region where the feed port 4 for feeding the crystal-containing slurry to the ripening tank is present is 4.

Regarding the inner diameter perpendicular to the partition when the ripening tank is viewed from the upper side, in the two regions, the ratio of the length of an inner-diameter portion in the region where the withdrawal port 12 for withdrawing the mother liquor from the ripening tank is present to the length of an inner-diameter portion in the region where the feed port 4 for feeding the crystal-containing slurry to the ripening tank is present is 2.9.

The distance from the partition 5 to the baffle 2a or 2b is 72 mm.

The horizontal distance from the feed port 4 to the baffle 2b is 170 mm.

The horizontal distance from the separation mechanism 6 to the baffle 2a is 24 mm.

The horizontal distance from the slurry withdrawal port 20 to the baffle 2a is 14 mm.

The horizontal distance from the feed port 4 for feeding the crystal-containing slurry to the ripening tank to the withdrawal port 12 for withdrawing the mother liquor from the ripening tank is 208 mm.

### (Example 1)

A ripening tank of Example 1 was operated, and the slurry containing acrylic acid crystals in the ripening tank was agitated by the agitator to generate a swirling flow. The swirling flow collided with the baffles to generate an upward flow. As a result, a supernatant part consisting of a supernatant and a suspension part where acrylic acid crystals were suspended were formed.

The ratio of the volume of the suspension part to the total volume of the inside of the ripening tank, which was taken as 1, was 2/3. The ratio of the volume of the supernatant part to the total volume of the inside of the ripening tank, which was taken as 1, was 1/3. The suspension state in the suspension part was uniform, and no substances such as crystals were deposited on the bottom of the ripening tank.

Further, the mother liquor was able to be suitably withdrawn and recovered from the supernatant part, while the crystals in the fed slurry were not mixed into the mother liquor, achieving continuous normal operation. This enabled crystals to be suitably grown in the ripening tank.

### (Comparative Example 1)

A ripening tank was operated as in Example 1, except that no baffle was provided in the ripening tank. The slurry containing acrylic acid crystals in the ripening tank was agitated by the agitator to generate a swirling flow. Yet, the swirling flow was a co-flow in which no upward or downward liquid flow occurred, and a rotating vortex was created around the agitator shaft, resulting in uneven agitation. As a result, a supernatant part was not formed, crystals were deposited on the bottom of the ripening tank. Further, when the mother liquor is withdrawn, crystals in the fed slurry were mixed into the mother liquor, making normal operation difficult.

### (Example 2)

A ripening tank was operated as in Example 1, except that no partition was provided in the ripening tank. The slurry containing acrylic acid crystals in the ripening tank was agitated by the agitator to generate a swirling flow. The swirling flow collided with the baffle to generate an upward flow. As a result, a supernatant part consisting of a supernatant and a suspension part where acrylic acid crystals were suspended were formed.

The suspension state in the suspension part was uniform, and no substances such as crystals were deposited on the bottom of the ripening tank. Although the interface between the supernatant part and the suspension part was slightly disturbed, and when the mother liquor was withdrawn, a small amount the crystals in the fed slurry were mixed into the mother liquor, continuous normal operation was achieved. This enabled crystals to be suitably grown in the ripening tank.

### (Example 3)

A ripening tank was operated as in Example 1, except that no separating mechanism was provided in the ripening tank. The slurry containing acrylic acid crystals in the ripening tank was agitated by the agitator to generate a swirling flow. The swirling flow collided with the baffle to generate an upward flow. As a result, a supernatant part consisting of a supernatant and a suspension part where acrylic acid crystals were suspended were formed.

The suspension state in the suspension part was uniform, and no substances such as crystals were deposited on the bottom of the ripening tank. Although when the mother liquor was withdrawn, a small amount of the crystals in the fed slurry were mixed into the mother liquor, continuous normal operation was achieved. This enabled crystals to be suitably grown in the ripening tank.

### (Example 4)

A ripening tank was operated as in Example 1, except that the nozzle constituting the slurry withdrawal port was provided in parallel to the normal direction of the contour line corresponding to the inner wall surface of the ripening tank when the ripening tank was viewed from the upper side, and the slurry was withdrawn not by the tangential withdrawing, but by side-wall withdrawing in which the slurry was withdrawn in the normal direction. The slurry containing acrylic acid crystals in the ripening tank was agitated by the agitator to generate a swirling flow. The swirling flow collided with the baffles to generate an upward flow. As a result, a supernatant part consisting of a supernatant and a suspension part where acrylic acid crystals were suspended were formed.

Although the suspension state in the suspension part was almost uniform, a small amount of crystals were deposited on the bottom of the ripening tank.

Further, the mother liquor was able to be suitably withdrawn and recovered from the supernatant part, while the crystals in the fed slurry were not mixed into the mother liquor, achieving continuous normal operation. This enabled crystals to be suitably grown in the ripening tank.

The tangential withdrawing in Examples 1 to 3 is a method of withdrawing the slurry, using a nozzle, along the tangential direction of the contour line corresponding to the inner wall surface of the ripening tank when the ripening tank is viewed from the upper side. The side-wall withdrawing in Example 4 is a method of withdrawing the slurry, using a nozzle, along the normal direction of the contour line corresponding to the inner wall surface of the ripening tank when the ripening tank is viewed from the upper side.

The results of Examples 1 to 4 demonstrate that when the ripening tank capable of keeping crystals of a compound suspended in the ripening tank includes an agitator and a baffle for generating an upward flow from a swirling flow and is capable of forming a supernatant part consisting of a supernatant and a suspension part where the crystals of a compound are suspended, the following can be achieved. Specifically, the suspension state in the suspension part can be made sufficiently uniform, and coarse crystals can be sufficiently prevented from depositing on the bottom of the ripening tank. Also, crystals can be sufficiently prevented from mixing the supernatant part, achieving continuous normal operation. As a result, crystals can be grown suitably in the ripening tank, and a high-quality product can be obtained.

### REFERENCE SIGNS LIST

1: (ripening) tank
2a, 2b: baffle
3: agitator blade
4: nozzle (feed port) (for feeding crystal-containing slurry to (ripening tank))
5: partition
6: separation mechanism
7: supernatant part
8: suspension part
9a: agitating direction
9b: flow of crystal-containing slurry
11a: slurry containing crystals of compound (to be fed)
12: withdrawal port (for mother liquor)
13, 15: mother liquor
20: withdrawal port (for withdrawing crystal-containing slurry from (ripening) tank)
21: crystal-containing slurry (to be withdrawn)
112a: nozzle (constituting mother-liquor withdrawal port)
114a: nozzle (constituting extra withdrawal port for withdrawing mother liquor from (ripening) tank)
120b: nozzle (constituting withdrawal port for crystal-containing slurry)
d: vertical distance

## Claims

1. A tank for use in a purification apparatus, the tank being at least one of a crystallization tank that forms a slurry containing crystals of a compound or a ripening tank that is capable of keeping crystals of a compound suspended in the ripening tank,
the tank including:
an agitator; and
a baffle for generating an upward flow from a swirling flow,
the tank being capable of forming a supernatant part consisting of a supernatant and a suspension part where the crystals of a compound are suspended.

2. The tank according to claim 1, further comprising, above a top of the baffle:
a feed port for feeding a slurry containing crystals of a compound to the tank;
a withdrawal port for withdrawing a mother liquor from the tank; and
a partition provided between the feed port and the withdrawal port along a direction from a top-roof side to a bottom side of the tank.

3. The tank according to claim 1, further comprising, above a top of the baffle:
a withdrawal port for withdrawing a mother liquor from the tank; and
a separation mechanism including a weir for overflowing the supernatant and flows the supernatant into the withdrawal port so as to prevent the crystals of a compound from mixing into the mother liquor.

4. The tank according to any one of claims 1 to 3, further comprising, below the top of the baffle: a withdrawal port for withdrawing the slurry containing crystals of a compound from the tank,
the withdrawal port being provided so as to withdraw the slurry containing crystals of a compound along a tangential direction of a contour line corresponding to an inner wall surface of the tank when the tank is viewed from an upper side.

5. The tank according to claim 3, further comprising, above the withdrawal port for withdrawing the mother liquor from the tank: an extra withdrawal port for withdrawing the mother liquor from the tank,
the extra withdrawal port being provided such that a position in height of a lower end of an opening of the extra withdrawal port is equal to or lower than a position in height of a top of the weir in the separation mechanism.

6. The tank according to any one of claims 1 to 5,
wherein the baffle is provided in the vicinity of the inner wall surface of the tank.

7. A purification apparatus comprising the tank according to any one of claims 1 to 6.

8. A method for producing a compound, the method comprising:
feeding a slurry containing crystals of a compound to a tank;
agitating the slurry containing crystals of a compound in the tank; and
withdrawing the slurry containing crystals of a compound agitated in the agitating from the tank,
the tank being at least one of a crystallization tank that forms a slurry containing crystals of a compound or a ripening tank that is capable of keeping crystals of a compound suspended in the ripening tank,
the tank including:
an agitator; and
a baffle for generating an upward flow from a swirling flow,
the tank being capable of forming a supernatant part consisting of a supernatant and a suspension part where the crystals of a compound are suspended.

9. The method for producing a compound according to claim 8,
wherein the compound is a (meth)acrylic acid.

10. The method for producing a compound according to claim 9,
wherein the (meth)acrylic acid is produced from a raw material including at least one selected from the group consisting of propane, propylene, acrolein, isobutene, methacrolein, acetic acid, lactic acid, isopropanol, 1,3-propanediol, glycerol, and 3-hydroxypropionic acid.

11. A method for purifying a compound, the method comprising:
feeding a slurry containing crystals of a compound to a tank;
agitating the slurry containing crystals of a compound in the tank; and
withdrawing the slurry containing crystals of a compound agitated in the agitating from the tank,
the tank being at least one of a crystallization tank that forms a slurry containing crystals of a compound or a ripening tank that is capable of keeping crystals of a compound suspended in the ripening tank,
the tank including:
an agitator; and
a baffle for generating an upward flow from a swirling flow,
the tank being capable of forming a supernatant part consisting of a supernatant and a suspension part where the crystals of a compound are suspended.
